# EUROPÄISCHE PATENTSCHRIFT

(11) **EP 4 257 081 B1**
(45) Veröffentlichungstag und Bekanntmachung des Hinweises auf die Patenterteilung: **18.09.2024**
(21) Anmeldenummer: 23166207.3
(22) Anmeldetag: 31.03.2023
(51) Int. Cl.: A61F 2/16

(54) **OPHTHALMOLOGISCHES IMPLANTAT MIT EINEM GRUNDKÖRPER UND EINEM KRAFTSENSOR SOWIE VORRICHTUNG ZUM ERMITTELN EINER KRAFT, WELCHE AUF EIN OPHTHALMOLOGISCHES IMPLANTAT EINWIRKT**
OPHTHALMOLOGICAL IMPLANT COMPRISING A BASE BODY AND A FORCE SENSOR AND DEVICE FOR DETERMINING A FORCE ACTING ON AN OPHTHALMOLOGICAL IMPLANT
IMPLANT OPHTALMOLOGIQUE COMPRENANT UN CORPS DE BASE ET UN CAPTEUR DE FORCE ET DISPOSITIF DE DÉTERMINATION D'UNE FORCE AGISSANT SUR UN IMPLANT OPHTALMOLOGIQUE

(30) Priorität: 08.04.2022 DE 102022108611
(43) Veröffentlichungstag der Anmeldung: 11.10.2023
(73) Patentinhaber: Carl Zeiss Meditec AG, 07745 Jena (DE)
(72) Erfinder: NICOLI, Francesca, 73447 Oberkochen (DE); BADUR, Thorben, 73447 Oberkochen (DE)
(74) Vertreter: Hofstetter, Schurack & Partner

(56) Entgegenhaltungen:
- US-A- 6 096 078
- US-A1- 2017 079 771
- US-A1- 2018 023 942
- US-B2- 7 926 940

## Beschreibung

### Technisches Gebiet

Die Erfindung betrifft ein ophthalmologisches Implantat mit einem Grundkörper und einem Kraftsensor sowie eine Vorrichtung zum Ermitteln einer Kraft, welche auf zumindest einen Bereich eines Grundkörpers eines solchen ophthalmologischen Implantats einwirkt.

### Stand der Technik

Ophthalmologische Implantate unterliegen während der Implantation unterschiedlichen Kräften. Beispielsweise wird durch den Kapselsack Kraft auf eine zu implantierende Intraokularlinse ausgeübt. Es ist daher wichtig, die auftretenden Kräfte in vivo zu überwachen, um Beschädigungen des Gewebes oder des Implantats zu verhindern. Die gegenwärtig vorgeschlagenen Möglichkeiten zur Überwachung solcher Kräfte bestehen in der Verwendung mechanischer oder elektronischer Sensoren am Implantat. Beispielsweise beschreibt die US 2015/0276372 A1 die Verwendung von amorphen Kohlenstoffschichten als Dehnungssensoren. Um das elektrische Signal in diesen Kohlenstoffschichten auszulesen, sind aber entsprechende Steckverbindungen und Energieversorgungsleitungen erforderlich. Die bisherigen Möglichkeiten zur Ermittlung von auf ein Implantat einwirkenden Kräften stehen damit vor großen Herausforderungen in Bezug auf Miniaturisierung, Biokompatibilität, Signalauswertung und Handhabung und stoßen in der Praxis an Grenzen.

Ein ophthalmologisches Implantat mit einem Grundkörper und einem Kraftsensor ist aus dem Dokument US-A-2018/023942 bekannt.

### Darstellung der Erfindung

Aufgabe der vorliegenden Erfindung ist es, ein ophthalmologisches Implantat zu schaffen, welches eine einfachere Ermittlung und Überwachung von auf das Implantat wirkenden Kräften erlaubt. Eine weitere Aufgabe der Erfindung ist es, eine geeignete Vorrichtung zum Ermitteln einer Kraft, die auf ein solches ophthalmologisches Implantat einwirkt, zu schaffen.

Die Aufgabe wird erfindungsgemäß durch ein ophthalmologisches Implantat gemäß Patentanspruch 1 sowie durch eine Vorrichtung mit den Merkmalen des Patentanspruchs 10 gelöst. Vorteilhafte Ausgestaltungen mit zweckmäßigen Ausbildungen der Erfindung sind in den Unteransprüchen angegeben, wobei vorteilhafte Ausgestaltungen des ophthalmologischen Implantats als vorteilhafte Ausgestaltungen der Vorrichtung anzusehen sind.

Ein erster Aspekt der Erfindung betrifft ein ophthalmologisches Implantat mit einem Grundkörper und einem Kraftsensor, wobei der Kraftsensor zumindest teilweise aus wenigstens einem Interferenzfarbstoff besteht, welcher seinen Farbeindruck in Abhängigkeit von einer auf zumindest einen Bereich des Grundkörpers einwirkenden Kraft verändert. Mit anderen Worten ist es erfindungsgemäß vorgesehen, dass der Kraftsensor teilweise oder vollständig aus einem Interferenzfarbstoff gebildet ist, der seine Farbe ändert, wenn sich die Materialbelastung des Grundkörpers durch eine einwirkende Kraft ändert, beispielsweise bei dehnenden und/oder stauchenden Verformungen des Grundkörpers. Unter einem Interferenzfarbstoff wird im Rahmen der vorliegenden Offenbarung eine Verbindung verstanden, welche aufgrund ihrer Struktur bei gewissen Wellenlängen, die im einfallenden Lichte vertreten sind, durch Interferenz wechselwirkt. Das physikalische Prinzip hinter Strukturfarben ist also nicht die Absorption, sondern die Beugung. Dieses Phänomen tritt auf, wenn Licht bzw. elektromagnetische Strahlung mit periodischen Strukturelementen des Interferenzfarbstoffs in Wechselwirkung tritt, wobei der Abstand (Periodizität) zwischen den Strukturelementen des Interferenzfarbstoffs in der gleichen Größenordnung wie die einfallende(n) Wellenlänge(n) liegt. Die spezifische(n) Wellenlänge(n), die diese Abstandsbedingung(en) erfüllt bzw. erfüllen, wird bzw. werden von den periodischen Strukturelementen gestreut, wodurch die Wellen konstruktiv und/oder destruktiv überlagern. Das resultierende Licht wird vom Interferenzfarbstoff reflektiert und kann als Farbeindruck wahrgenommen werden. Die Bedingung der konstruktiven Interferenz hängt dabei vom Abstand der Strukturelemente (d), der Wellenlänge des einfallenden Lichts (λ) und dem Einfallswinkel (θ) ab, für den die konstruktive Interferenz am stärksten ist. Dieser Zusammenhang kann mit der Formel nλ = 2d sin θ (Bragg'sche Beugung) beschrieben werden, in welcher n eine natürliche Zahl ist, die die Beugungsordnung angibt. Der Ursprung der Farbe liegt in diesem Fall also nicht in einem spezifischen elektronischen Übergang in den Molekülen wie beispielsweise bei Fluoreszenzfarbstoffen, sondern in einer bestimmten räumlichen Organisation der Strukturelemente des Interferenzfarbstoffs, die damit als eine Art Gitter wirken. Durch das Einwirken einer Kraft auf den Interferenzfarbstoff verändert sich der Abstand bzw. die Periodizität seiner beugenden Strukturen, was die Farbverschiebung verursacht und entsprechend detektiert werden kann. Ein Interferenzfarbstoff bietet damit erhebliche Vorteile gegenüber herkömmlichen Absorptions- bzw. Fluoreszenzfarbstoffen, da diese aufgrund der Empfindlichkeit des Moleküls im angeregten Zustand gegenüber oxidativem Stress einem chemischen Abbau unterliegen. Dagegen bietet die Verwendung eines Interferenzfarbstoffs eine deutlich höhere Gestaltungsfreiheit und eine gleichbleibende Komplexität, die nahezu unabhängig von der gewählten Detektions- bzw. Zielwellenlänge ist. Zudem sind Interferenzfarbstoffe wesentlich photostabiler als Absorptions- bzw. Fluoreszenzfarbstoffe und können sehr präzise an gewünschte Wellenlängen bzw. Wellenlängenbereiche angepasst werden.

Die Farbe eines Interferenzfarbstoffs hängt wie bereits beschrieben wesentlich von der Periodizität seiner Strukturelemente ab. Wenn die Periodizität verändert wird, verschiebt sich auch die reflektierte Wellenlänge. Dieses Prinzip kann im Rahmen der vorliegenden Erfindung zur Detektion von einwirkenden Kräften genutzt werden. Die durch die einwirkende Kraft bzw. die einwirkenden Kräfte verursachte(n) Änderung(en) der Periodizität führt zu korrespondierenden Wellenlägenverschiebung(en) des reflektierten Lichts. Derartige Wellenlägenverschiebungen können dann mit geringem apparativem Aufwand berührungslos gemessen werden und erlauben Rückschlüsse auf die verursachende, auf das Implantat wirkende Kraft. Mit anderen Worten kann das "Signal" des Kraftsensors, das heißt der durch die einwirkende Kraft verursachte Farbwechsel des reflektierten Lichts, rein optisch und damit berührungslos ermittelt werden. Der Kraftsensor kann dabei im Bereich von wenigen kPa präzise ansprechen.

Durch die einfache Anpassbarkeit von Interferenzfarbstoffen kann der Kraftsensor zudem besonders einfach an eine gewünschte Wellenlänge bzw. einen gewünschten Wellenlängenbereich oder an mehrere Wellenlängenbereiche sowie an unterschiedlich große zu erwartende Kräfte angepasst werden. Damit ist es zum Beispiel möglich, eine normale weiße Lichtquelle zur Beaufschlagung des Kraftsensors zu verwenden und das von diesem reflektierte Licht mit einer einfachen Kamera zu erfassen und weiter zu prozessieren. Schließlich kann auch die Position des Kraftsensors auf dem Implantat besonders flexibel gewählt werden, zumal keinerlei elektrischen oder mechanischen Anschluss- oder Versorgungsleitungen benötigt werden. Insbesondere kommt der Kraftsensor ohne bewegliche oder elektronische Komponenten sowie ohne Stromzufuhr aus. Vorzugsweise wird der Kraftsensor ausschließlich aus einem oder mehreren Interferenzfarbstoffen, die auch als strukturelle Farben bezeichnet werden, gebildet. Alternativ kann vorgesehen sein, dass der Kraftsensor eine oder mehrere weitere Verbindungen, die keine strukturelle Farbe ist bzw. sind, umfasst.

Als Struktur- bzw. Interferenzfarbstoffe können grundsätzlich alle geeigneten Verbindungen, die gegebenenfalls als Nanopartikel vorliegen, verwendet werden. Generell eignen sich viele Nanopartikel, da sie vergleichsweise einfach zu synthetisieren sind und aufgrund ihrer geometrischen Eigenschaften eine einfache Abstimmung auf bestimmte Wellenlängen ermöglichen. Bei der Verwendung von nicht oder eingeschränkt biokompatiblen Nanopartikeln, beispielsweise Metall- oder Halbleiterpartikeln, kann die Biokompatibilität beispielsweise durch Immobilisierung und/oder Einkapselung verbessert werden.

Der wenigstens eine Kraftsensor des erfindungsgemäßen ophthalmologischen Implantats ist dabei vorzugsweise maschinenlesbar und damit auch maschinenauswertbar ausgebildet. Hierdurch kann ein maschineller Ermittlungs- und Prüfprozess durchgeführt werden, wodurch eine zuverlässigere und komfortablere Handhabung des erfindungsgemäßen Implantats ermöglicht ist und eine Überbelastung des Implantats oder des Gewebes, eine Fehlstellung des Implantats und dergleichen zuverlässig verhindert werden können. Beispielsweise kann der Kraftsensor mit einem einfachen Operationsmikroskop ausgelesen werden, ohne dass wie bei Fluoreszenzfarbstoffen ein Laser mit speziellen Wellenlängen verwendet werden muss. In weiterer Ausgestaltung kann ein geeigneter, an sich bekannter Polarisator im optischen Ausleseweg angeordnet werden.

Weiterhin kann vorgesehen sein, dass ein Kraftsensor mindestens zwei Interferenzfarbstoffe mit unterschiedlichen Farben umfasst. Unter Interferenzfarbstoffen mit unterschiedlichen Farben sind dabei Interferenzfarbstoffe zu verstehen, die aufgrund ihres unterschiedlichen strukturellen Aufbaus mit unterschiedlichen Wellenlängen des eingestrahlten Lichts wechselwirken und damit unter ansonsten gleichen oder vergleichbaren Beleuchtungsbedingungen zu unterschiedlichen Farbeindrücken führen. Dies stellt eine besonders vorteilhafte Möglichkeit zur Detektion von unterschiedlich starken Kräften bzw. von unterschiedlich starken Auswirkungen einer auf den Grundkörper wirkenden Kraft auf unterschiedliche Bereiche des Grundkörpers dar, da nicht nur die relativen Positionen der unterschiedlichen Interferenzfarbstoffe bezüglich des Grundkörpers, sondern auch deren jeweilige Farben bzw. kraftbedingte Farbänderungen ermittelt und ausgewertet werden können. Ebenso kann generell vorgesehen sein, dass der wenigstens eine Interferenzfarbstoff in Form eines Oberflächenmusters auf dem Grundkörper angeordnet ist. Hierdurch kann neben einer krafteinwirkungsbedingten Farbänderung auch eine Änderung der Geometrie des Oberflächenmusters ermittelt und zur Auswertung und Analyse der einwirkenden Kraft bzw. Kräfte herangezogen werden.

In einer besonders vorteilhaften Ausgestaltung der Erfindung ist vorgesehen, dass der wenigstens eine Interferenzfarbstoff zumindest teilweise oder vollständig aus Nanocellulose, insbesondere Zellulosenanokristalle (CNC, NCC), Zellulosenanofasern (CNF), nanofibrillierte Zellulose (NFC), cholesterischer Nanocellulose und/oder bakterielle Nanocellulose, besteht. Besonders bevorzugt sind dabei cholesterische Zellulosenanokristalle und/oder Zellulosenanofasern. Nanocellulose ist ein Material mit besonders vorteilhaften optischen, mechanischen und chemischen Eigenschaften. Cellulose-Nanokristalle (CNCs) wurden als verantwortlich für die Erzeugung der leuchtenden, schillernden Farben von verschiedenen Pflanzen entdeckt. Dabei sind die Farben wie bereits beschrieben nicht auf das Vorhandensein eines bestimmten Pigments zurückzuführen, sondern auf die Wechselwirkung zwischen dem Licht und den periodischen Mikrostrukturen der Nanocellulose in Blütenblättern und Blättern, weshalb Interferenzfarben auch "Strukturfarben" genannt werden. Zellulose ist an sich zudem ein biokompatibles Material biologischen Ursprungs, das vom menschlichen Körper abgebaut und daher problemlos und normalerweise ohne weitere Zusatzbehandlungen wie Immobilisierung oder Einkapselung zur teilweisen oder vollständigen Realisierung des Kraftsensors verwendet werden kann. Zellulose ist weithin kostengünstig und aus nachhaltigen Quellen verfügbar. Mit der Verwendung von Nanocellulose entfällt im Vergleich zu Absorptions- und Fluoreszenzfarbstoffen zudem die Notwendigkeit von speziellen Lese- oder Auswertegeräten. Nanocellulose kann zudem als Folie ausgebildet werden. Die mechanische Flexibilität der Nanocellulosefolie ermöglicht die besonders einfache Integration dieser Technologie auf und/oder in einem Grundkörper des erfindungsgemäßen Implantats. Mit anderen Worten kann die Nanocellulosefolie als Beschichtung verwendet oder auch in das LOL-Material eingebettet werden. Indem sich aufgrund einer Krafteinwirkung die Helixsteigung der cholesterischen Struktur der cholesterischen Zellulosenanokristalle ändert, wird die Farbverschiebung verursacht. Durch Druck nähern sich die Steigungen der Helix an und verändern so die strukturelle Periodizität und damit die Färbung. Umgekehrt wächst durch Zug der Abstand der Helices bzw. schraubenförmigen Strukturen, wodurch sich ebenfalls die strukturelle Periodizität und damit die Färbung des Interferenzfarbstoffes ändern. Generell gilt dabei, dass sich das Maximum des Reflexionsvermögens umso stärker verschiebt je geringer der Abstand zwischen den Zellulosenanokristallen im Ausgangszustand ist. Ein Konglomerat aus selbstorganisierten cholesterischen Zellulosenanokristallen kann die gleichen Abmessungen wie der gesamte Kraftsensor besitzen. Das bedeutet, dass das Konglomerat so groß wie die vom Kraftsensor abgedeckte Fläche ist. Vorzugsweise bestehen die Zellulosenanokristalle aus Partikeln bzw. Partikelkonglomeraten mit einer Länge von mindestens 50 µm oder mehr.

In einer besonders vorteilhaften Ausgestaltung der Erfindung besteht die Nanocellulose zumindest anteilig oder vollständig aus Hydroxypropylcellulose. Hydroxypropylcellulose (HPC) ist ein Sammelbegriff für Celluloseether, bei denen ein Teil der Hydroxygruppen mit Hydroxypropyl-Gruppen verethert sind. HPCs sind biokompatibel, pH-stabil und gehören zudem zu den thermoresponsiven Polymeren, wodurch sie grundsätzlich nicht nur zur Detektion von Krafteinwirkungen, sondern auch zur Detektion von Temperaturänderungen verwendet werden können. Weiterhin können die gewünschten Interferenzfarbeigenschaften von HPC-Nanocellulose besonders einfach variiert und eingestellt werden.

In einer vorteilhaften Ausgestaltung der Erfindung ist vorgesehen, dass der wenigstens eine Interferenzfarbstoff Partikel und/oder Partikelkonglomerate enthält, die eine mittlere Größe von mindestens 40 µm, insbesondere von mindestens 50 µm besitzen, um einen Farbeindruck im für den Menschen sichtbaren Wellenlängenbereich zwischen etwa 380 nm und etwa 750 nm zu erzeugen. Mit anderen Worten ist es vorgesehen, dass Interferenzfarbstoff Partikel aufweist, deren Größe im angegebenen Nanometerbereich liegt und/oder die in entsprechend großen Konglomeraten, das heißt in größeren Ansammlungen oder "Baugruppen" angeordnet sind. Ein Partikelkonglomerat ("Baugruppe") enthält also mehrere, in einer bestimmten Struktur zusammengelagerte Partikel, beispielsweise Zellulose-Nanokristallgruppen. Die Partikel und/oder Partikelkonglomerate haben eine Mindestgröße von 40 µm, können aber auch größer sein. Diese Mindestgröße der Partikel bzw. Partikelkonglomerate gewährleistet eine ausreichende Periodizität der Struktur, um mit einstrahlendem Licht im für den Menschen sichtbaren Wellenlängenbereich konstruktiv interferieren zu können.

Alternativ oder zusätzlich ist es vorgesehen, dass der Interferenzfarbstoff selbstorganisationsfähige Partikel und/oder Partikelkonglomerate umfasst, die dazu ausgebildet sind, sic0h in einer geordneten, insbesondere periodischen Struktur anzuordnen. Hierdurch erfolgt automatisch eine für den gewünschten Farbeindruck benötigte Ausrichtung und Anordnung der einzelnen Partikel bzw. Strukturelemente des Interferenzfarbstoffs beim Auftragen auf den Grundkörper. Im Rahmen der vorliegenden Offenbarung werden mit dem Begriff "Partikel" generell auch Fasern bezeichnet, bei welchen es sich um Partikel mit einer länglichen Form handelt. Weiterhin kann der Begriff "Partikel" im Rahmen der vorliegenden Offenbarung auch unrunde, ovale und unregelmäßig geformte Strukturelemente bezeichnen.

Weitere Vorteile ergeben sich dadurch, dass der wenigstens eine Interferenzfarbstoff auf dem Grundkörper und/oder innerhalb des Grundkörpers und/oder auf einer Beschichtung des Grundkörpers und/oder innerhalb einer Beschichtung des Grundkörpers und/oder unter einer Beschichtung des Grundkörpers angeordnet ist. Hierdurch kann der Interferenzfarbstoff besonders flexibel angeordnet werden, um den Kraftsensor zu realisieren. Insbesondere wenn der wenigstens eine Interferenzfarbstoff innerhalb des Grundkörpers, innerhalb einer Beschichtung des Grundkörpers und/oder unter einer Beschichtung des Grundkörpers angeordnet ist, ist dabei ein besonders hoher Schutz vor mechanischer Beschädigung gegeben, wodurch der Kraftsensor besonders gut vor Umwelteinflüssen geschützt ist und auch nach der ursprünglichen Operation noch zuverlässig ausgelesen werden kann, beispielsweise um die korrekte Anordnung und Ausrichtung des Implantats zu kontrollieren. Die Beschichtung kann vorzugsweise als Schutzschicht ausgebildet sein und/oder besonders bevorzugt einen Brechungsindex aufweisen, der zumindest im Wesentlichen dem des Kammerwassers entspricht. Hierzu eignen sich beispielsweise fluorierte oder perfluorierte (Meth)Acrylate. Auf diese Weise können unerwünschte optische Brechungseffekte an den Phasengrenzen vermieden werden. Bedarfsweise kann der Interferenzfarbstoff auch kovalent an das Material des Grundkörpers und/oder der Beschichtung gebunden sein. Ebenso kann der Interferenzfarbstoff in Abhängigkeit von seiner Ausgestaltung als Folie ausgebildet sein und damit selbst eine Beschichtung bilden.

Weitere Vorteile ergeben sich dadurch, dass der wenigstens eine Interferenzfarbstoff mit Licht im Wellenlängenbereich zwischen 300 nm und 3000 nm, insbesondere im Wellenlängenbereich zwischen 400 nm und 750 nm und/oder im Wellenlängenbereich zwischen etwa 750 nm und etwa 3000 nm, wechselwirkt. Eine besonders einfache Ermittlung und Auswertung des "Signals" des Kraftsensors ist dabei möglich, wenn der wenigstens eine Interferenzfarbstoff durch Wechselwirkung mit Licht im Wellenlängenbereich zwischen 300 nm und 900 nm, insbesondere im für den Menschen sichtbaren Wellenlängenbereich zwischen etwa 400 nm und 750 nm, einen Farbeindruck erzeugt. Grundsätzlich können aber auch Interferenzfarbstoffe mit einem hohen Reflexionsgrad im nahen Infrarotbereich zwischen etwa 750 nm bis etwa 3000 nm sehr vorteilhaft sein, insbesondere da außerhalb des für den Menschen wahrnehmbaren Wellenlängenbereichs keine Gefahr einer optischen Beeinträchtigung für den Patienten besteht.

In einer weiteren vorteilhaften Ausgestaltung der Erfindung ist vorgesehen, dass das ophthalmologische Implantat als Kapselspannring oder als Stent oder als Intraokularlinse, insbesondere als modulare Intraokularlinse und/oder akkommodierende Intraokularlinse, ausgebildet ist. Hierdurch können die erfindungsgemäßen Vorteile für unterschiedliche Implantattypen realisiert werden. Das Implantat kann dabei grundsätzlich mehrteilig bzw. modular ausgebildet sein und mehrere Grundkörper, beispielsweise eine Frontlinse und eine Basislinse, umfassen, die zusammengesetzt werden.

Weitere Vorteile ergeben sich dadurch, dass der Grundkörper wenigstens einen optischen Teil und wenigstens einen haptischen Teil umfasst, wobei der haptische Teil vorzugsweise gewölbt ist. Durch das Vorhandensein eines haptischen Teils kann der optische Teil ohne Kraftsensor ausgebildet werden, um optische Störungen zu vermeiden. Vor allem eine gewölbte Haptik kann von Vorteil sein, da sie unter Druck eine Verschiebung der axialen Position des Implantats verursachen.

Weitere Vorteile ergeben sich dadurch, dass der Grundkörper wenigstens einen optischen und wenigstens einen haptischen Teil umfasst, wobei der Kraftsensor auf dem wenigstens einen optischen Teil und/oder auf dem wenigstens einen haptischen Teil angeordnet ist. Hierdurch ist eine besonders hohe Freiheit bei der Anordnung des Kraftsensors gegeben. Der Kraftsensor kann grundsätzlich auch aus mehreren Teilen oder Bereichen bestehen, die an jeweils unterschiedlichen Stellen des Grundkörpers angeordnet sind. Ebenso kann der Kraftsensor teilweise oder vollständig in einem Übergangsbereich zwischen dem optischen und dem haptischen Teil angeordnet sein. Diese Konfiguration kann dem Chirurgen während des Eingriffs helfen, die Platzierung des Implantats besser zu bestimmen, indem er überwacht, wie der Druck am Übergang verteilt ist. So lässt sich beispielsweise feststellen, wie stark der optische Teil von der haptischen Ebene abweicht und in welche Richtung das Implantat verschoben werden muss, um eine sofortige Korrektur der Brechungsfehler zu ermöglichen. Vorzugsweise ist der Kraftsensor nicht in einer zentralen optischen Zone des optischen Teils angeordnet. Die zentrale optische Zone ist für Intraokularlinsen definiert als der zentrale Bereich mit einem Durchmesser von 4,4 mm. In diesem Bereich ist ein Kraftsensor nach dem derzeitigen Stand nicht ISOkonform. Der Kraftsensor kann aber in der peripheren optischen Zone, das heißt im äußeren Bereich des optischen Teils eines ophthalmologischen Implantats angeordnet sein, wodurch die zentrale optische Zone mit einem Durchmesser von 4,4 mm frei bleibt. In diesem peripheren Bereich ist ein Kraftsensor nach dem derzeitigen Stand ISOkonform. Mit Hilfe eines im Bereich der optischen Zone angeordneten Kraftsensors kann beispielsweise die Linsenkrümmung einer IOL gemessen bzw. eingestellt werden. Die Möglichkeit, die vom Gewebe, beispielsweise dem Kapselsack, auf den haptischen Teil ausgeübten Kräfte zu messen, ist in vielen Anwendungsfällen ebenfalls von grundlegender Bedeutung für die zuverlässige Ermittlung der axialen Platzierung des Implantats im Gewebe und der Stabilität des Implantats in Bezug auf Neigung und Zentrierung. Vorzugsweise ist der Kraftsensor generell in dem Bereich des Grundkörpers angeordnet, der erwartungsgemäß während der Operation am stärksten belastet wird. Die Messung der Kraft, welcher ein haptischer Teil ausgesetzt ist, kann also helfen, das Implantat besser im Gewebe zu platzieren und damit ein besseres refraktives Ergebnis durch den optischen Teil für den Patienten zu erzielen.

Ein zweiter Aspekt der Erfindung betrifft eine Vorrichtung zum Ermitteln einer Kraft, welche auf zumindest einen Bereich eines Grundkörpers eines ophthalmologischen Implantats gemäß dem ersten Erfindungsaspekt einwirkt. Die erfindungsgemäße Vorrichtung eignet sich zum Ermitteln einer Kraft, die auf ein solches ophthalmologisches Implantat einwirkt, dadurch, dass sie eine Erfassungseinrichtung, mittels welcher eine den Farbeindruck des Kraftsensors charakterisierende Farbinformation ermittelbar ist, und eine Auswertungseinrichtung, welche zum Datenaustausch mit der Erfassungseinrichtung gekoppelt ist und dazu ausgebildet ist, anhand der Farbinformation die zumindest auf den Bereich des Grundkörpers des ophthalmologischen Implantats wirkende Kraft zu ermitteln, umfasst. Mit anderen Worten weist die Vorrichtung mindestens eine Erfassungseinrichtung, beispielsweise eine Kamera, auf, mittels welcher die durch Krafteinwirkung verursachten Farbänderungen des Interferenzfarbstoffs des Kraftsensors des ophthalmologischen Implantats ermittelbar sind, sowie eine mit der Erfassungseinrichtung gekoppelte Auswertungseinrichtung auf, welche dazu ausgebildet ist, anhand der ermittelten Farbinformationen die auf den Grundkörper des Implantats wirkende(n) Kraft bzw. Kräfte zu ermitteln. Für den Fall, dass der wenigstens eine Interferenzfarbstoff des Kraftsensors in Form eines Oberflächenmusters auf dem Grundkörper angeordnet ist, kann neben einer krafteinwirkungsbedingten Farbänderung auch eine Änderung der Geometrie des Oberflächenmusters ermittelt und zur Auswertung und Analyse der einwirkenden Kraft bzw. Kräfte herangezogen werden. Falls das Implantat zwei oder mehr Kraftsensoren aufweist, besteht zusätzlich die Möglichkeit, Kraftverteilungen auf der Grundlage der Unterschiede zwischen den verschiedenen Farbinformationen der einzelnen Kraftsensoren zu ermitteln.

Die erfindungsgemäße Vorrichtung kann über eine optionale Mensch-Maschine-Schnittstelle sofort eine akustische, optische und/oder haptische Rückmeldung über die momentan einwirkenden Kräfte geben und beispielsweise Informationen über den Zustand von spannungsempfindlichen Teilen des Implantats liefern. Die Vorrichtung kann auch verwendet werden, um die Krümmungsänderung von akkommodierenden Intraokularlinsen während der Implantation zu überwachen und zu steuern. Weiterhin kann die Vorrichtung auch als Werkzeug zur Kalibrierung anderer Geräte verwendet werden.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: eine schematische Ansicht eines ophthalmologischen Implantats gemäß einer ersten Ausführungsform;
- Fig. 2: eine schematische Ansicht des ophthalmologischen Implantats gemäß einer zweiten Ausführungsform;
- Fig. 3: eine schematische Seitenansicht des ophthalmologischen Implantats gemäß einer dritten Ausführungsform;
- Fig. 4: eine schematische Seitenansicht des ophthalmologischen Implantats gemäß einer vierten Ausführungsform;
- Fig. 5: eine schematische Ansicht einer Frontlinse des ophthalmologischen Implantats gemäß einer fünften Ausführungsform;
- Fig. 6: eine schematische Ansicht einer Basislinse des ophthalmologischen Implantats gemäß der fünften Ausführungsform;
- Fig. 7: eine schematische Ansicht der zusammengesetzten Frontlinse und Basislinse des ophthalmologischen Implantats gemäß der fünften Ausführungsform;
- Fig. 8: eine perspektivische und teilgeschnittene Ansicht eines Kraftsensors, der auf einem ophthalmologischen Implantat zur Überwachung der Linsenkrümmung angeordnet ist; und
- Fig. 9: eine schematische Schnittansicht einer akkommodierenden Intraokularlinse mit beispielhaft angeordneten Kraftsensoren.

### Bevorzugte Ausführung der Erfindung

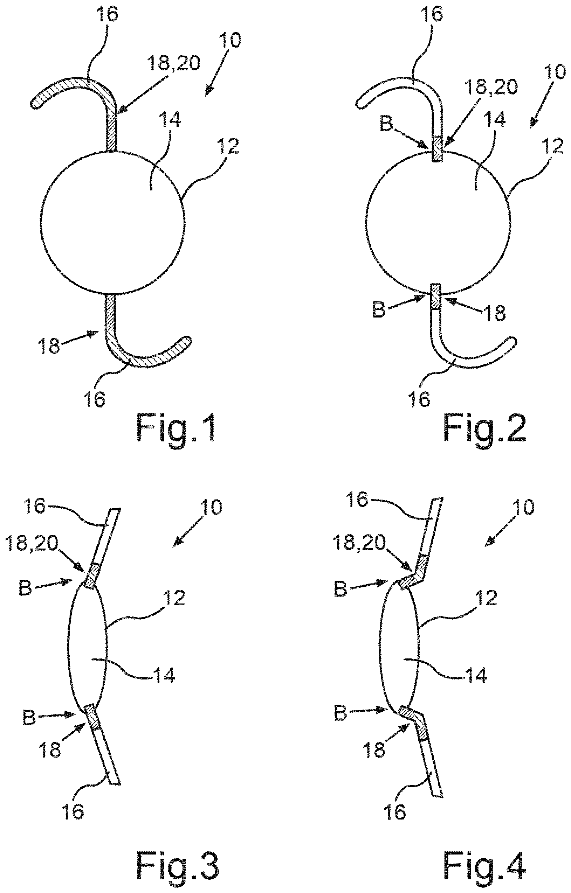

Fig. 1 zeigt eine schematische Ansicht eines ophthalmologischen Implantats 10 gemäß einer ersten Ausführungsform. Das ophthalmologische Implantat 10, welches vorliegend als Intraokularlinse (IOL) ausgebildet ist, weist einen Grundkörper 12 mit einem optischen Teil 14 und zwei haptischen Teilen 16 auf. Im vorliegenden Ausführungsbeispiel weisen beide haptischen Teile 16 jeweils einen Kraftsensor 18 auf, wobei jeder Kraftsensor 18 sich zumindest im Wesentlichen über den gesamten jeweiligen haptischen Teil 16 erstreckt und jeweils aus einem Interferenzfarbstoff 20 besteht, welcher seinen Farbeindruck in Abhängigkeit von einer auf zumindest einen Bereich des Grundkörpers 12 einwirkenden Kraft verändert. Der Interferenzfarbstoff 20 besteht jeweils aus einer druckempfindlichen Folie aus cholesterischen Hydroxypropylcellulose-Nanokristallen (CNC), mit welcher der Grundkörper 12 beschichtet ist. Die CNC-Nanokristalle bilden dabei Partikelkonglomerate, die eine mittlere Größe von mindestens 40 µm, insbesondere von mindestens 50 µm besitzen. Die mechanische Flexibilität der Zellulosefolie 20 ermöglicht die Integration des Kraftsensors 18 auf einer IOL 10. Die Nanozellulosefolie 20 kann als Beschichtung verwendet oder auch in das Material des Grundkörpers 12 eingebettet werden. Die Dicke des Kraftsensors 18 kann variieren, ist aber vorzugsweise dünn genug, um die mechanischen und optischen Eigenschaften des Implantats 10 nicht nachteilig zu verändern. Der Kraftsensor 18 kann generell in verschiedenen Größen und Formen hergestellt werden, je nach Anwendungsbereich.

Indem die gesamte Haptik 16 mit dieser druckempfindlichen CNC-Folie 20 als Kraftsensor 18 beschichtet ist, kann die durch unterschiedliche Schraffierung angedeutete Kraftverteilung entlang der gesamten Oberfläche der haptischen Teile 16 während der Operation überwacht werden. Der Kraftsensor 18 fungiert damit auch als eine Art Dehnungssensor, da er neben punktuellen Krafteinwirkungen insbesondere auch dehnende und stauchende Verformungen durch entsprechende Farbänderung anzeigt. Die Möglichkeit, die vom Kapselsack auf die Haptik 16 ausgeübten Kräfte zu messen, ist von grundlegender Bedeutung für die korrekte axiale Platzierung der IOL 10 im Kapselsack und die Stabilität der IOL 10 in Bezug auf Kippung und Dezentrierung. Die Interferenzfarbstoffe 20 wechselwirken vorliegend mit Licht im Wellenlängenbereich zwischen 400 nm und 750 nm, das heißt im für den Menschen sichtbaren Bereich. Damit kann die Farbänderung durch Einstrahlung von weißem Licht, also beispielsweise durch herkömmliche Lampen, beispielsweise Beleuchtungseinheiten eines Operationsmikroskops, sichtbar gemacht werden. Die Farbe der Interferenzfarbstoffe 20, die grundsätzlich gleich oder unterschiedlich ausgebildet sein können, hängt wesentlich von der Periodizität ihrer Strukturelemente ab. Wenn die Periodizität verändert wird, verschiebt sich auch die reflektierte Wellenlänge. Dieses Prinzip wird im Rahmen der vorliegenden Erfindung zur Detektion von einwirkenden Kräften genutzt. Die durch die einwirkende Kraft bzw. die einwirkenden Kräfte verursachte(n) Änderung(en) der Periodizität führt zu korrespondierenden Wellenlägenverschiebung(en) des reflektierten Lichts. Derartige Wellenlägenverschiebungen können dann mit geringem apparativem Aufwand berührungslos gemessen werden und erlauben Rückschlüsse auf die verursachende, auf das Implantat 10 wirkende Kraft. Mit anderen Worten kann das "Signal" des Kraftsensors 18, das heißt der durch die einwirkende Kraft verursachte Farbwechsel des reflektierten Lichts, rein optisch und damit berührungslos ermittelt werden. Der Kraftsensor 18 kann dabei im Bereich von wenigen kPa präzise ansprechen und apparativ mit einer entsprechenden Vorrichtung überwacht werden. Die Vorrichtung (nicht gezeigt) benötigt dazu in einfachster Ausgestaltung lediglich eine Erfassungseinrichtung, mittels welcher eine den Farbeindruck des Kraftsensors 18 charakterisierende Farbinformation ermittelbar ist, und eine Auswertungseinrichtung, welche zum Datenaustausch mit der Erfassungseinrichtung gekoppelt ist und dazu ausgebildet ist, anhand der Farbinformation die zumindest auf den Bereich des Grundkörpers 12 des ophthalmologischen Implantats 10 wirkende Kraft zu ermitteln. Falls das Implantat zwei oder mehr Kraftsensoren 18 aufweist, besteht zusätzlich die Möglichkeit, Kraftverteilungen auf der Grundlage der Unterschiede zwischen den verschiedenen Farbinformationen der einzelnen Kraftsensoren 18 zu ermitteln.

Fig. 2 zeigt eine schematische Ansicht des ophthalmologischen Implantats 10 gemäß einer zweiten Ausführungsform, während Fig. 3 zur weiteren Verdeutlichung eine schematische Seitenansicht des ophthalmologischen Implantats 10 gemäß einer dritten Ausführungsform zeigt. Der grundlegende Aufbau des Implantats 10 entspricht dabei der ersten Ausführungsform. Im Unterschied zur ersten Ausführungsform sind die Kraftsensoren 18 im zweiten und dritten Ausführungsbeispiel aber nicht entlang der gesamten Oberfläche der haptischen Teile 16, sondern lediglich in jeweiligen Übergangsbereichen B zwischen dem optischen Teil 14 und den beiden haptischen Teilen 16 angeordnet.

Fig. 4 zeigt eine schematische Seitenansicht des ophthalmologischen Implantats 10 gemäß einer vierten Ausführungsform. Im Unterschied zu den vorhergehenden Ausführungsformen sind die haptischen Teile 16 gewölbt bzw. geknickt ausgebildet und verlaufen nicht in einer Ebene. Auch in diesem Beispiel sind die Kraftsensoren 18 lediglich in den Übergangsbereichen B vom optischen Teil 14 zu den haptischen Teilen 16 angeordnet. Dies ist vor allem bei den gezeigten gewölbten bzw. geknickten Haptiken 16 von Vorteil, da diese unter Druck eine Verschiebung der axialen Position der IOL 10 verursachen. Die Messung der Kraft, der die haptischen Teile 16 ausgesetzt sind, kann daher helfen, die IOL 10 besser zu positionieren und ein besseres Ergebnis für den Patienten zu erzielen.

Fig. 2 stellt damit eine Prinzipdarstellung von Ausführungsformen dar, bei denen der Kraftsensor 18 im Übergangsbereich B zwischen dem optischen Teil 14 und den haptischen Teilen 16 angeordnet ist. Bei Fig. 3 und Fig. 4 handelt es sich um Ausführungsformen, die spezielle haptische Geometrien aufweisen (Fig. 3: abgewinkelt, Fig. 4: stufenförmig). Diese beiden in Fig. 3 und Fig. 4 gezeigten Ausführungsformen sind besonders gut geeignet, um mit Hilfe des Kraftsensors 18 eine mögliche axiale Verschiebung des Implantats 10 zu detektieren.

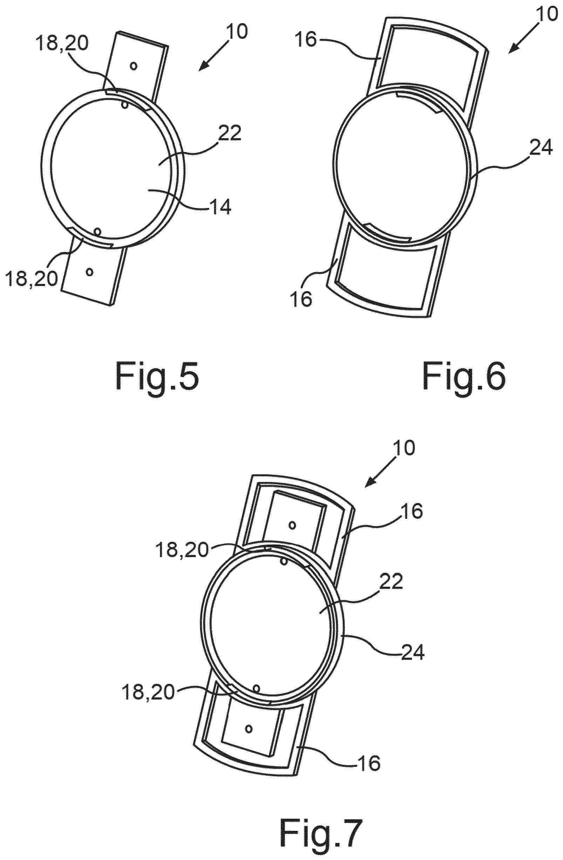

Fig. 5 zeigt eine schematische Ansicht einer Frontlinse 22 des ophthalmologischen Implantats 10 gemäß einer fünften Ausführungsform. Fig. 5 wird in Zusammenschau mit Fig. 6 und Fig. 7 erläutert, wobei Fig. 6 eine schematische Ansicht einer Basislinse 24 des ophthalmologischen Implantats gemäß der fünften Ausführungsform und Fig. 7 eine schematische Ansicht der zusammengesetzten Frontlinse 22 und Basislinse 24 des ophthalmologischen Implantats 10 gemäß der fünften Ausführungsform zeigen. Man erkennt, dass an den beiden Verbindungsstellen zwischen dem durch die Frontlinse 22 gebildeten optischen Teil 14 und dem durch die Basislinse 24 gebildeten haptischen Teil 16 jeweilige Kraftsensoren 18 aus Interferenzfarbstoffen 20 angeordnet sind, um an diesen Schnittstellen zwischen den beiden Linsenmodulen 22, 24 ein spannungsfreies Einsetzen der modularen Frontlinse 22 in die modulare Basislinse 24 sicherzustellen. Alternativ oder zusätzlich ist es möglich, dass die Basislinse 24 über einen oder mehrere Kraftsensoren 18 verfügt.

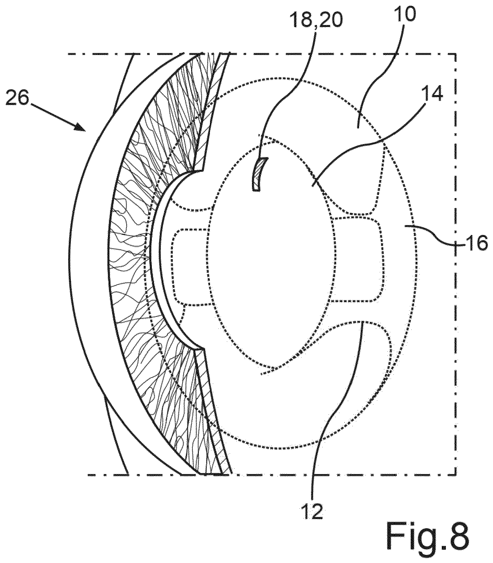

Fig. 8 zeigt eine perspektivische und teilgeschnittene Ansicht eines Kraftsensors 18, der auf einem als Intraokularlinse (IOL) ausgebildeten ophthalmologischen Implantat 10 zur direkten Überwachung der Linsenkrümmung angeordnet ist. Das Implantat 10 ist dabei im implantierten Zustand in ein Auge 26 eines Patienten gezeigt. Der optische Teil 14 der IOL 10 ist flüssigkeitsgefüllt. Die Linsenkrümmung kann mit Hilfe des auf dem optischen Teil 14 aber außerhalb der peripheren optischen Zone angeordneten Kraftsensors 18, der wiederum einen CNC-Interferenzfarbstoff 20 umfasst bzw. aus diesem besteht, überwacht und präzise eingestellt werden. Eine Krümmungsänderung führt dabei zu einer entsprechenden Farbänderung des Interferenzfarbstoffs 20, die mit einer entsprechenden Vorrichtung überwacht und als Steuerparameter zur Steuerung und/oder Regelung der Linsenkrümmung verwendet werden kann.

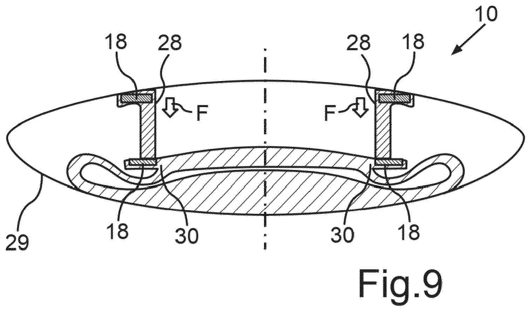

Fig. 9 zeigt eine schematische Schnittansicht einer akkommodierenden Intraokularlinse 10, deren grundsätzlicher Aufbau aus der WO 2020/025325 A1 bekannt ist und welche erfindungsgemäß mit mehreren Kraftsensoren 18 versehen ist. Die Kraftsensoren 18 bestehen aus cholesterischen CNCs und sind an beispielhaften Positionen der Intraokularlinse 10 angeordnet. Kraftsensoren 18 können beispielsweise an bzw. in einem Aktuator 28 angeordnet sein, der nach der Implantation in Kontakt mit dem Kapselsack 29 steht, so dass die Kräfte F gemessen werden können, die der Kapselsack auf den Aktuator 28 ausübt. Kraftsensoren 18 können zusätzlich oder alternativ an einem Biegeelement oder einer Formanpassungseinrichtung 30 der Intraokularlinse 10 angeordnet sein, welche mit dem Aktuator 28 in Kontakt stehen bzw. von diesem betätigt werden und die Formveränderung der Intraokularlinse 10 bewirken. Anstelle der vier gezeigten Kraftsensoren 18 können auch mehr oder weniger Kraftsensoren 18 vorgesehen sein. Weiterhin können ein oder mehrere Kraftsensoren 18 an einer anderen Stelle der IOL 10 angeordnet sein. Die verwendeten CNCs sind selbstorganisierend und ordnen sich automatisch in der cholesterischen Anordnung an. Eine Farbverschiebung des Kraftsensors 18 bei Krafteinwirkung ist dann das Ergebnis einer Änderung der Helixsteigung der cholesterischen Struktur. Durch den zusätzlichen Druck nähern sich die Steigungen der Helices an und verändern so die strukturelle Periodizität und damit die Färbung des Kraftsensors 18. Umgekehrt vergrößern sich durch eine Zugbelastung die Steigungen der Helices, was zu einer entsprechenden Farbänderung des Kraftsensors 18 führt.

Der erfindungsgemäße Kraftsensor 18 kann generell auch bei anderen Implantattypen, beispielsweise bei Kapselspannringen oder Stents zur Kraft- bzw. Dehnungs-/Stauchungsmessung verwendet werden.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, DIN-Toleranzen und dergleichen - als vom Rahmen der Erfindung mitumfasst anzusehen.

### Bezugszeichenliste

- 10: Implantat
- 12: Grundkörper
- 14: optischer Teil
- 16: haptischer Teil
- 18: Kraftsensor
- 20: Interferenzfarbstoff
- 22: Frontlinse
- 24: Basislinse
- 26: Auge
- 28: Aktuator
- 29: Kapselsack
- 30: Formanpassungseinrichtung
- B: Übergangsbereich
- F: Kraft

## Patentansprüche

1. Ophthalmologisches Implantat (10) mit einem Grundkörper (12) und einem Kraftsensor (18), **dadurch gekennzeichnet, dass** der Kraftsensor (18) zumindest teilweise aus wenigstens einem Interferenzfarbstoff (20) besteht, welcher seinen Farbeindruck in Abhängigkeit von einer auf zumindest einen Bereich des Grundkörpers (12) einwirkenden Kraft verändert.

2. Ophthalmologisches Implantat (10) nach Anspruch 1,
**dadurch gekennzeichnet, dass**
der wenigstens eine Interferenzfarbstoff (20) zumindest teilweise aus Nanocellulose, insbesondere Zellulosenanokristalle, Zellulosenanofasern, nanofibrillierter Zellulose, cholesterischer Nanocellulose und/oder bakterieller Nanocellulose, besteht.

3. Ophthalmologisches Implantat (10) nach Anspruch 2,
**dadurch gekennzeichnet, dass**
die Nanocellulose zumindest anteilig aus Hydroxypropylcellulose besteht.

4. Ophthalmologisches Implantat (10) nach einem der Ansprüche 1 bis 3,
**dadurch gekennzeichnet, dass**
der wenigstens eine Interferenzfarbstoff (20) Partikel und/oder Partikelkonglomerate enthält, die eine mittlere Größe von mindestens 40 µm, insbesondere von mindestens 50 µm besitzen, und/oder dass der Interferenzfarbstoff (20) selbstorganisationsfähige Partikel und/oder Partikelkonglomerate umfasst, die dazu ausgebildet sind, sich in einer geordneten, insbesondere periodischen Struktur anzuordnen.

5. Ophthalmologisches Implantat (10) nach einem der Ansprüche 1 bis 4,
**dadurch gekennzeichnet, dass**
der wenigstens eine Interferenzfarbstoff (20) auf dem Grundkörper (12) und/oder innerhalb des Grundkörpers (12) und/oder auf einer Beschichtung des Grundkörpers (12) und/oder innerhalb einer Beschichtung des Grundkörpers (12) und/oder unter einer Beschichtung des Grundkörpers (12) angeordnet ist.

6. Ophthalmologisches Implantat (10) nach einem der Ansprüche 1 bis 5,
**dadurch gekennzeichnet, dass**
der wenigstens eine Interferenzfarbstoff (20) mit Licht im Wellenlängenbereich zwischen 300 nm und 3000 nm, insbesondere im Wellenlängenbereich zwischen 400 nm und 750 nm und/oder im Wellenlängenbereich zwischen etwa 750 nm und etwa 3000 nm, wechselwirkt.

7. Ophthalmologisches Implantat (10) nach einem der Ansprüche 1 bis 6,
**dadurch gekennzeichnet, dass**
dieses als Kapselspannring oder als Stent oder als Intraokularlinse, insbesondere als modulare Intraokularlinse und/oder akkommodierende Intraokularlinse ausgebildet ist.

8. Ophthalmologisches Implantat (10) nach einem der Ansprüche 1 bis 7,
**dadurch gekennzeichnet, dass**
der Grundkörper (12) wenigstens einen optischen Teil (14) und wenigstens einen haptischen Teil (16) umfasst, wobei der haptische Teil (16) vorzugsweise gewölbt ist.

9. Ophthalmologisches Implantat (10) nach Anspruch 8,
**dadurch gekennzeichnet, dass**
der Kraftsensor (18) auf dem wenigstens einen optischen Teil (14) und/oder auf dem wenigstens einen haptischen Teil (16) und/oder in einem Übergangsbereich (B) von wenigstens einem optischen Teil (14) zu wenigstens einem haptischen Teil (16) angeordnet ist.

10. Vorrichtung zum Ermitteln einer Kraft, welche auf zumindest einen Bereich eines Grundkörpers (12) eines ophthalmologischen Implantats (10) nach einem der Ansprüche 1 bis 9 einwirkt, umfassend:
- eine Erfassungseinrichtung, mittels welcher eine den Farbeindruck des Kraftsensors (18) charakterisierende Farbinformation ermittelbar ist; und
- eine Auswertungseinrichtung, welche zum Datenaustausch mit der Erfassungseinrichtung gekoppelt ist und dazu ausgebildet ist, anhand der Farbinformation die zumindest auf den Bereich des Grundkörpers (12) des ophthalmologischen Implantats (10) wirkende Kraft zu ermitteln.

## Claims

1. Ophthalmological implant (10) having a main body (12) and a force sensor (18),
**characterized in that**
the force sensor (18) at least partly consists of at least one interference dye (20) which changes its colour impression depending on a force acting on at least one region of the main body (12).

2. Ophthalmological implant (10) according to Claim 1, **characterized in that**
the at least one interference dye (20) at least partly consists of nanocellulose, in particular cellulose nanocrystals, cellulose nanofibres, nanofibrillated cellulose, cholesteric nanocellulose and/or bacterial nanocellulose.

3. Ophthalmological implant (10) according to Claim 2, **characterized in that**
the nanocellulose at least proportionally consists of hydroxypropylcellulose.

4. Ophthalmological implant (10) according to any of Claims 1 to 3,
**characterized in that**
the at least one interference dye (20) contains particles and/or particle conglomerates which have a mean size of at least 40 pm, in particular of at least 50 pm, and/or in that the interference dye (20) comprises self-assemblable particles and/or particle conglomerates which are designed to arrange themselves in an ordered, in particular periodic, structure.

5. Ophthalmological implant (10) according to any of Claims 1 to 4,
**characterized in that**
the at least one interference dye (20) is arranged on the main body (12) and/or within the main body (12) and/or on a coating of the main body (12) and/or within a coating of the main body (12) and/or beneath a coating of the main body (12).

6. Ophthalmological implant (10) according to any of Claims 1 to 5,
**characterized in that**
the at least one interference dye (20) interacts with light in the wavelength range of between 300 nm and 3000 nm, in particular in the wavelength range of between 400 nm and 750 nm and/or in the wavelength range of between approximately 750 nm and approximately 3000 nm.

7. Ophthalmological implant (10) according to any of Claims 1 to 6,
**characterized in that**
this implant is designed as a capsular tension ring or as a stent or as an intraocular lens, in particular as a modular intraocular lens and/or an accommodating intraocular lens.

8. Ophthalmological implant (10) according to any of Claims 1 to 7,
**characterized in that**
the main body (12) comprises at least one optical part (14) and at least one haptic part (16), the haptic part (16) preferably being curved.

9. Ophthalmological implant (10) according to Claim 8, **characterized in that**
the force sensor (18) is arranged on the at least one optical part (14) and/or on the at least one haptic part (16) and/or in a transition region (B) from at least one optical part (14) to at least one haptic part (16).

10. Apparatus for determining a force which acts on at least one region of a main body (12) of an ophthalmological implant (10) according to any of Claims 1 to 9, comprising:
- a capture device, by means of which colour information characterizing the colour impression of the force sensor (18) is determinable; and
- an evaluation device, which is coupled to the capture device for data exchange and is designed to determine the force acting at least on the region of the main body (12) of the ophthalmological implant (10) on the basis of the colour information.

## Revendications

1. Implant ophtalmologique (10), comprenant un corps de base (12) et un capteur de force (18),
**caractérisé en ce que** le capteur de force (18) est composé au moins partiellement d'au moins un colorant d'interférence (20) qui modifie son impression de couleur en fonction d'une force agissant sur au moins une zone du corps de base (12).

2. Implant ophtalmologique (10) selon la revendication 1, **caractérisé en ce que** ledit au moins un colorant d'interférence (20) est composé au moins partiellement de nanocellulose, en particulier de nanocristaux de cellulose, de nanofibres de cellulose, de cellulose nanofibrillée, de nanocellulose cholestérique et/ou de nanocellulose bactérienne.

3. Implant ophtalmologique (10) selon la revendication 2, **caractérisé en ce que** la nanocellulose est composée au moins proportionnellement de hydroxypropylcellulose.

4. Implant ophtalmologique (10) selon l'une quelconque des revendications 1 à 3, **caractérisé en ce que** ledit au moins un colorant d'interférence (20) contient des particules et/ou des conglomérats de particules qui possèdent une taille moyenne d'au moins 40 pm, en particulier d'au moins 50 pm, et/ou **en ce que** le colorant d'interférence (20) comprend des particules et/ou conglomérats de particules capables de s'auto-organiser, qui sont réalisés pour s'arranger dans une structure ordonnée, en particulier périodique.

5. Implant ophtalmologique (10) selon l'une quelconque des revendications 1 à 4, **caractérisé en ce que** ledit au moins un colorant d'interférence (20) est disposé sur le corps de base (12) et/ou à l'intérieur du corps de base (12) et/ou sur un revêtement du corps de base (12) et/ou à l'intérieur d'un revêtement du corps de base (12) et/ou sous un revêtement du corps de base (12).

6. Implant ophtalmologique (10) selon l'une quelconque des revendications 1 à 5, **caractérisé en ce que** ledit au moins un colorant d'interférence (20) interagit avec une lumière dans la plage de longueurs d'onde comprise entre 300 nm et 3000 nm, en particulier dans la plage de longueurs d'onde comprise entre 400 nm et 750 nm et/ou dans la plage de longueurs d'onde comprise entre environ 750 nm et environ 3000 nm.

7. Implant ophtalmologique (10) selon l'une quelconque des revendications précédentes 1 à 6, **caractérisé en ce qu'**il est réalisé sous forme d'anneau de tension capsulaire ou de stent ou de lentille intraoculaire, en particulier sous forme de lentille intraoculaire modulaire et/ou de lentille intraoculaire d'accommodation.

8. Implant ophtalmologique (10) selon l'une quelconque des revendications 1 à 7, **caractérisé en ce que** le corps de base (12) comprend au moins une partie optique (14) et au moins une partie haptique (16), dans lequel la partie haptique (16) est de préférence courbe.

9. Implant ophtalmologique (10) selon la revendication 8, **caractérisé en ce que** le capteur de force (18) est disposé sur ladite au moins une partie optique (14) et/ou sur ladite au moins une partie haptique (16) et/ou dans une zone de transition (B) d'au moins une partie optique (14) à au moins une partie haptique (16).

10. Dispositif permettant d'établir une force qui agit sur au moins une zone d'un corps de base (12) d'un implant ophtalmologique (10) selon l'une quelconque des revendications 1 à 9, comprenant :
- un dispositif de détection au moyen duquel une information de couleur caractérisant l'impression de couleur du capteur de force (18) peut être établie ; et
- un dispositif d'évaluation qui est couplé au dispositif de détection pour l'échange de données et qui est réalisé pour établir à l'aide de l'information de couleur la force agissant au moins sur la zone du corps de base (12) de l'implant ophtalmologique (10).
